# EUROPEAN PATENT APPLICATION

(11) **EP 2 845 901 A1**
(43) Date of publication of application: **11.03.2015**
(21) Application number: 13777591.2
(22) Date of filing: 12.04.2013
(51) Int. Cl.: C12N 15/09, A01H 5/00

(54) **NOVEL CAMPANULA FLAVONOID 3',5'-HYDROXYLASE GENE AND USE OF SAME**

(30) Priority: 16.04.2012 JP 2012092716
(71) Applicant: Suntory Holdings Limited, Osaka-shi, Osaka 530-8203 (JP)
(72) Inventor: TANAKA, Yoshikazu, Mishima-gun Osaka 618-8503 (JP); KATSUMOTO, Yukihisa, Mishima-gun Osaka 618-8503 (JP)
(74) Representative: Keirstead, Tanis Evelyne
(86) International application number: PCT/JP2013/061116
(87) International publication number: WO 2013/157502

(57) **Abstract**

There is provided a novel Campanula flavonoid 3',5'-hydroxylase gene, and a plasmid comprising the gene under the control of the cauliflower mosaic virus 35S promoter.

## Description

### Technical Field

The present invention relates to a genetic engineering technique wherein an exogenous gene is introduced into plant cells. More specifically, the invention relates to a novel Campanula flavonoid 3',5'-hydroxylase gene, to a plasmid containing the gene under the control of the cauliflower mosaic virus 35S promoter, and to a method for producing a garden rose plant with notably increased delphinidin content in the petals by transformation using the plasmid by the *Agrobacterium* method.

### Background Art

Plant cross-breeding methods include (i) hybridization by crossing of stamen and pistil, (ii) natural or artificial mutation, and (iii) gene recombination. Of these methods, gene recombinant technology may be utilized to impart new traits to plants by expressing useful genes in target plants, without being constrained by the genetic restrictions of the species. A wide variety of gene recombinant plants created in this manner are already being cultivated throughout the world.

Flower color components include anthocyanins, carotenoids and betalains. Anthocyanins are secondary metabolite members of the general group of compounds called flavonoids, and they are synthesized from phenylalanine by the action of numerous enzymes. The color of an anthocyanin depends on its structure. Specifically, the color is bluer with increasing numbers of hydroxyl groups on the anthocyanidin B-ring, which is the chromophoric group of the anthocyanin. The major anthocyanidins, delphinidin, cyanidin and pelargonidin in that order, have increasing number of hydroxyl groups on the B-ring. Anthocyanins derived from delphinidin accumulate in most blue flowers. Also, it is known that increasing numbers of aromatic acyl groups (coumaroyl groups, caffeoyl groups and the like) modifying anthocyanins produce increasing blue color for anthocyanins (that is, the absorption maximum is shifted to the long wavelength end), which corresponds to increased stability of the anthocyanins. In particular, anthocyanins with multiple bonded aromatic acyl groups are known as polyacylated anthocyanins, which are present in blue petals of gentian, clitoria and the like (see Plant J. 54, 737-749, 2008). Enzymes involved in the biosynthesis of anthocyanins, and the genes coding for them, have already been isolated from numerous plants (see Plant J. 54, 737-749, 2008). Anthocyanin colors depend on the local pH of vacuoles, and on the presence of other flavonoids, metal ions and the like. Specifically, anthocyanins exhibit red color at low vacuole pH, and blue color at neutrality. Flavones and flavonols are referred to as copigments, and these have effects of causing anthocyanins to appear blue when they are copresent with anthocyanins. Furthermore, it is known that iron and aluminum ion coordinate with the hydroxyl groups of the anthocyanin B-ring, rendering the anthocyanin blue.

Plants bloom with a large array of different flower colors, but there is no variety that can bloom with all flower colors. This is because the pigments that can be synthesized by a variety are genetically determined. For example, roses, carnations, chrysanthemums, lilies, gerberas and the like have no natural blue or violet varieties. The major reason for this is that these plants lack a gene for flavonoid 3',5'-hydroxylase (hereunder, "F3'5'H") which is necessary for synthesis of delphinidin. Several reports have documented attempts to express the F3'5'H gene in petals in order to produce delphinidin and produce a blue flower color. Such research is based on the importance of selecting an appropriate promoter and an appropriate F3'5'H gene for each variety, as described below, in order to increase the delphinidin content of the petals and shift the flower color toward the blue end.

Expressing the F3'5'H gene in petunias, which lack F3'5'H, increases the level of anthocyanins derived from delphinidin (Nature, 1993, 366, 276-279, FEBS Lett. 1999, 461, 241-245). Also, expressing the F3'5'H gene in tobacco *(Nicotiana tabacum),* which stores cyanidin, results in synthesis of delphinidin and imparts a slightly blue flower color (FEBS Lett. 1999, 461, 241-245).

NPL 1 teaches that when F3'5'H genes derived from Campanula, bluebell and petunia were expressed in tobacco under the control of the cauliflower mosaic virus 35S promoter, the most efficient production of delphinidin was obtained by the Campanula F3'5'H gene (see Abstract of the publication).

Also, PTL 1 describes introducing the petunia-derived F3'5'H gene into rose under the control of the cauliflower mosaic virus 35S promoter (see Example 9), but here the Campanula-derived F3'5'H gene was simply introduced into tobacco, whereas nothing is mentioned regarding production of delphinidin or change in flower color in the rose plant body or rose petal.

In addition, NPL 2 describes expression of the clitoria and verbena F3'5'H genes in verbena, stating that expression of the clitoria-derived gene resulted in greater delphinidin production and clearly altered flower color.

PTL 2 also states that with expression of F3'5'H genes derived from petunia, gentian, clitoria, cineria, bluebell, pansy and lavender in rose, expression of the pansy-derived F3'5'H gene under the control of the cauliflower mosaic virus 35S promoter or the rose-derived chalcone synthase gene promoter resulted in production of delphinidin at several tens of percent of the total anthocyanidins, while the pansy-derived F3'5'H gene satisfactorily functioned in rose under the control of the cauliflower mosaic virus 35S promoter or the rose-derived chalcone synthase gene promoter.

NPL 3 teaches that an enhancer-added cauliflower mosaic virus 35S (E12 35S) promoter produced expression of exogenous genes with greater efficiency than cauliflower mosaic virus 35S promoter. Rose having the pansy F3'5'H gene introduced under the control of E12 35S promoter produces delphinidin and has altered flower color. Depending on the host variety it is possible to obtain rose comprising delphinidin at 95% or greater of the total anthocyanidins and having flower color altered to violet, and one such variety is already being marketed (Variety Accession No. 20992).

Furthermore, PTL 3 teaches that in rose having introduced a cassette that transfers the pansy F3'5'H gene under the control of the E12 35S promoter, the iris dihydroflavonol 4-reductase (hereunder, "DFR") gene under the control of the E12 35S promoter, and rose DFR double stranded RNA, the delphinidin content was nearly 100% and flower color was altered in the introduced rose varieties, regardless of the variety.

In order to alter the flower color in a rose that has product value, it is naturally desirable to increase the delphinidin content in the petals, but it is also important not to adversely affect growth of the rose. This is also essential when cross-breeding is carried out using a high-delphinidin-expressing gene recombinant rose as the parent. In other words, for a viable garden rose variety it is necessary for the transformation plasmid to be one comprising a specific combination of a specific high-expression promoter allowing high expression of F3'5'H, and F3'5'H having a specific sequence, that allow the delphinidin content in the petals to be maximized.

Incidentally, PTL 4 describes transferring the pansy-derived F3'5'H gene into the chrysanthemum varieties Improved Reagan and Dark Splendid Reagan using the rose chalcone synthase promoter, whereby a gene recombinant chrysanthemum was obtained having delphinidin stored at 50% or greater and having the flower color altered to blue.

In PTL 5, when F3'5'H genes derived from Campanula, verbena, cineria, pansy, petunia and lobelia were expressed in the chrysanthemum variety 94-765, utilizing the chrysanthemum flavanone 3-hydroxylase gene-derived promoter and tobacco alcohol dehydrogenase gene 5'-untranslated region, the F3'5'H genes derived from campanula, verbena, cineria and pansy functioned relatively satisfactorily, producing delphinidin at 25% or greater of the total anthocyanidins. In the same publication it is stated that of these, the campanula-derived F3'5'H gene functions most satisfactorily in chrysanthemum, producing delphinidin at 50% or greater.

The Mac1 promoter, which is a constitutive promoter, the snapdragon-derived chalcone synthase gene promoter, which is a petal-specific promoter, and the petunia-derived chalcone synthase gene promoter, which is a petal-specific promoter, have been used for expression of the petunia F3'5'H gene in carnation. In this case, the two different petal-specific promoters produced larger quantities of delphinidin than the Mac1 promoter (see PTL 6). It was demonstrated that in carnation petals, the petunia DFR gene promoter or anthocyanidin synthase gene promoter are effective for transferring exogenous genes (see PTLs 7 and 8). As other petal-specific promoters there have been reported the pansy F3'5'H gene promoter (see PTL 9), the perilla anthocyanin 3-acyl group transferase gene promoter (see PTL 10) and the cineria F3'5'H gene promoter (see PTL 11).

As mentioned above, although the F3'5'H gene can be expressed in different plant varieties to produce delphinidin, it is difficult to predict which promoter should be used for expressing which plant-derived F3'5'H gene in the target plant, and in order to produce delphinidin at high efficiency, i.e. with a delphinidin content of 80% or greater, preferably 85% or greater, more preferably 90% or greater and even more preferably 95% or greater, of the total anthocyanidins, it is necessary to conduct trial and error or highly complex experimentation.

Furthermore, in order to alter flower color to blue it is generally necessary to increase the delphinidin content to 50% or greater, preferably 60% or greater, more preferably 70% or greater, more preferably 80% or greater, more preferably 85% or greater, more preferably 90% or greater, more preferably 95% or greater, more preferably 99% or greater and most preferably 100%, of the total anthocyanidins, and simply overexpressing the F3'5'H gene is usually insufficient for this purpose. In the case of carnation, for example, expressing the F3'5'H gene and the petunia DFR gene in white carnation, which lacks dihydroflavonol 4-reductase (DFR), results in about 100% delphinidin, yielding recombinant carnation with the flower color altered to blue (see PTL 12). Overexpression of the pansy-derived F3'5'H gene and the iris-derived DFR gene in rose, while suppressing expression of the rose DFR gene, has resulted in a delphinidin content of about 100% and yielded recombinant rose with flower color altered to blue (see PTL 3). Increase in delphinidin content has also been achieved by overexpression of the pansy-derived F3'5'H gene in chrysanthemum while suppressing expression of the chrysanthemum F3'H gene (see PTL 4).

### Citation List

### Patent Literature

[PTL 1] Japanese Patent Publication No. 3403196
(WO93/18155)
[PTL 2] WO2004/020637
[PTL 3] WO2005/017147
[PTL 4] WO2009/062253
[PTL 5] WO2010/122849
[PTL 6] WO94/28140
[PTL 7] WO96/036716
[PTL 8] WO2009/062259
[PTL 9] WO2010/122850
[PTL 10] PCT/JP2010/053886
[PTL 11] PCT/JP2010/070516
[PTL 12] WO06/36716

### [Non-patent literature]

[NPL 1] Biosci. Biotechnol. Biochem., 67(1), 161-165, 2003
[NPL 2] Plant Biotechnology 2006, 23,5-11
[NPL 3] Plant Cell Physiol. (1996) 37: 49-59

### DISCLOSURE OF THE INVENTION

### Problems to be Solved by the Invention

In light of these circumstances, the problem to be solved by the present invention is to provide a transformation plasmid comprising a specific combination of a specific high-expression promoter allowing high expression of F3'5'H, and F3'5'H having a specific sequence, that allow the delphinidin content in the petals to be maximized in a viable garden rose variety.

### Means for Solving the Problems

The present inventors have conducted much research and experimentation directed toward solving this problem, and as a result we have completed this invention upon finding that it is possible to obtain a transformed rose plant storing delphinidin at a high content in the petals, by transforming a specific variety of rose plant by the *Agrobacterium* method using a plasmid comprising the Campanula F3'5'H gene having a specific sequence, under the control of the cauliflower mosaic virus 35S promoter.

Specifically, the present invention is as follows.
[1] A polynucleotide selected from the group consisting of the following (a) to (d):
   (a) a polynucleotide comprising the nucleotide sequence listed as SEQ ID NO: 1;
   (b) a polynucleotide having at least 97% sequence identity with a polynucleotide comprising the nucleotide sequence listed as SEQ ID NO: 1 or its complementary nucleotide sequence, and encoding a protein having flavonoid 3',5'-hydroxylase activity,
   (c) a polynucleotide encoding a protein comprising the amino acid sequence listed as SEQ ID NO: 2; and
   (d) a polynucleotide encoding a protein having an amino acid sequence with at least 97% sequence identity with the amino acid sequence listed as SEQ ID NO: 2, and having flavonoid 3',5'-hydroxylase activity.

[2] A plasmid in which a polynucleotide according to [1] above is linked in a functional manner under the control of the cauliflower mosaic virus 35S promoter.

[3] A plasmid according to [2] above, wherein the *nos* terminator is further linked in a functional manner.

[4] A method for producing a transformed rose plant or its progeny having a delphinidin content of 80% or greater in the petals, by transformation of a garden rose variety *(Rosa hybrida*) by the *Agrobacterium* method using a plasmid according to [2] or [3] above.

[5] The method according to [4] above, wherein the delphinidin content in the petals is 85% or greater.

[6] The method according to [5] above, wherein the delphinidin content in the petals is 90% or greater.

[7] The method according to [6] above, wherein the delphinidin content in the petals is 95% or greater.

[8] A transformed rose plant or its progeny, produced by the method according to any one of [4] to [7] above.

### Effect of the Invention

By transformation using a plasmid containing the Campanula flavonoid 3',5'-hydroxylase gene of the invention under the control of the cauliflower mosaic virus 35S promoter by the *Agrobacterium* method, it is possible to notably increase the delphinidin content in the petals of garden rose varieties.

### Best Mode for Carrying Out the Invention

The polynucleotide of the invention (SEQ ID NO: 1) encodes Campanula-derived F3'5'H, and comprises 521 residues. Throughout the present specification, the term "polynucleotide" refers to DNA or RNA. The polynucleotide of the invention is not limited to comprising a polynucleotide with the nucleotide sequence listed as SEQ ID NO: 1 or encoding a protein comprising its corresponding amino acid sequence (SEQ ID NO: 2), and it may be selected from among polynucleotides comprising the nucleotide sequence or its complementary sequence, or polynucleotides encoding for proteins having specific sequence homology and preferably sequence identity, with the amino acid sequence, and having flavonoid 3',5'-hydroxylase activity. It is preferably a polynucleotide having at least 97% sequence identity.

The polypeptide of the invention is a DNA molecule that hybridizes with the nucleotide sequence listed as SEQ ID NO: 1 under stringent conditions and encodes a protein with flavonoid 3',5'-hydroxylase activity. Here, the term "polynucleotide that hybridizes under stringent conditions" as used throughout the present specification means a polynucleotide obtained, for example, by the colony hybridization method, plaque hybridization method or Southern hybridization method, using as the probe all or a portion of a polynucleotide comprising the nucleotide sequence listed as SEQ ID NO: 1 or its complementary nucleotide sequence. The hybridization method used may be, for example, the method described in "Sambrook & Russell, Molecular Cloning: A Laboratory Manual Vol. 3, Cold Spring Harbor, Laboratory Press 2001" or "Ausubel, Current Protocols in Molecular Biology, John Wiley & Sons 1987-1997".

The term "stringent conditions" used throughout the present specification may be low stringent conditions, moderately stringent conditions or high stringent conditions. Here, "low stringent conditions" are, for example, conditions of 5 x SSC, 5 x Denhardt solution, 0.5% SDS, 50% formamide, 32°C. Also, "moderately stringent conditions" are, for example, conditions of 5 x SSC, 5 x Denhardt solution, 0.5% SDS, 50% formamide, 42°C. "High stringent conditions" are, for example, conditions of 5 x SSC, 5 x Denhardt solution, 0.5% SDS, 50% formamide, 50°C, and especially 0.1 x SSC, 0.1% SDS, 65°C or 0.1 x SSC, 0.1% SDS, 70°C. Under such conditions it may be expected that DNA having high homology will be obtained more efficiently with increasing temperature. However, potential factors affecting the stringency of hybridization are many including temperature, probe concentration, probe length, ionic strength, time and salt concentration, and similar stringency conditions can be created by appropriate selection of these factors by a person skilled in the art.

The amino acid sequence encoded by the polynucleotide of the invention may have a deletion, substitution and/or addition of one or more amino acids, so long as flavonoid 3',5'-hydroxylase activity is maintained.

Throughout the present specification, the terms "identity" and "homology" mean for polypeptide sequences (or amino acid sequences) or polynucleotide sequences (or nucleotide sequences), the quantity (number) of amino acid residues or nucleotides composing them that can be determined as identical between the two chains, in terms of the mutual agreement between them, meaning the degree of sequence correlation between two polypeptide sequences or two polynucleotide sequences, and the "identity" and "homology" can be easily calculated. Numerous methods are known for measuring homology between two polynucleotide sequences or polypeptide sequences, and the terms "identity" and "homology" are well known to those skilled in the art (for example, see Lesk, A. M. (Ed.), Computational Molecular Biology, Oxford University Press, New York, (1988); Smith, D. W. (Ed.), Biocomputing: Informatics and Genome Projects, Academic Press, New York, (1993); Grifin, A. M. & Grifin, H. G. (Ed.), Computer Analysis of Sequence Data: Part I, Human Press, New Jersey, (1994); von Heinje, G., Sequence Analysis in Molecular Biology, Academic Press, New York, (1987); Gribskov, M. & Devereux, J. (Ed.), Sequence Analysis Primer, M-Stockton Press, New York, (1991) and elsewhere).

Also, the numerical values for "identity" and "homology" used in the present specification, unless otherwise specified, may be the numerical values calculated using a homology search program known to those skilled in the art, but they are preferably numerical values calculated using the ClustalW program of MacVector Application (version 9.5, Oxford Molecular Ltd., Oxford, England).

The polynucleotide (nucleic acid, gene) of the invention "encodes" a protein of interest. Here, "encodes" means that it allows expression of the protein of interest in a state in which it exhibits its activity. Also, the term "encodes" includes both encoding a structural sequence (exon) that is a continuous section of the protein of interest, and encoding via an intervening sequence (intron).

A gene having a natural nucleotide sequence can be obtained by analysis with a DNA sequencer, for example, as described in the examples below. Also, DNA encoding an enzyme having a modified amino acid sequence can be synthesized using common site-specific mutagenesis or PCR, based on DNA having the natural nucleotide sequence. For example, a DNA fragment to be modified may be obtained by restriction enzyme treatment of natural cDNA or genomic DNA, and used as template for site-specific mutagenesis or PCR using primers with the desired mutation, to obtain a DNA fragment having the desired modification. The DNA fragment having the mutation may then be linked with a DNA fragment encoding another portion of the target enzyme.

Alternatively, in order to obtain DNA encoding an enzyme comprising a shortened amino acid sequence, for example, an amino acid sequence that is longer than the target amino acid sequence, such as DNA encoding the full length amino acid sequence, is cut with a selected restriction enzyme, and if the resulting DNA fragment does not encode the entire target amino acid sequence, a DNA fragment comprising the missing portion of the sequence may be synthesized and linked with it.

The polynucleotide of the invention is inserted into a plasmid together with the cauliflower mosaic virus 35S promoter. The 35S promoter is preferably the E12 35S promoter (supra) having augmented expression by linkage of two enhancer sequences in tandem to the 35S promoter commonly used in plant transformants. The polynucleotide of the invention and the 35S promoter are linked in a functional manner, for example, with the polynucleotide of interest being situated downstream from the promoter. The plasmid of the invention is used for transformation of a garden rose variety.

For the purpose of the invention, the term "garden rose variety" refers to *Rosa* plants of the *Rosaceae* family (scientific name: *Rosa hybrida*) in terms of taxonomical classification. Garden rose varieties are largely classified as Hybrid Tea, Floribunda and Polyantha roses based on their tree form and flower size, with the major pigment (anthocyanin) in the petals of all lines being of two types, the cyanidin-type and pelargonidin-type. The invention is a technique for converting the major anthocyanin in rose petals to the delphinidin-type, and such varieties and lines are suitable for use. The invention can be applied for cyanidin-type varieties (for example, Cool Water, Ocean Song and Fame), or pelargonidin-type varieties (for example, Noblesse, Topless and Peach Avalanche).

The plasmid of the invention may further comprise non-promoter factors, for example, regulatory sequences such as terminators, polyadenylated signal sequences and replication origin sequences (ori); and selective markers such as drug resistance markers, fluorescent protein markers, enzyme markers and cell surface receptor markers. The terminator is preferably *nos* terminator, situated downstream from the polynucleotide of interest. Throughout the present specification, *"nos* terminator" refers to the terminator sequence of the nopaline synthase gene from the Ti plasmid of *Agrobacterium tumefaciens.*

### [Examples]

The present invention will now be explained in greater detail by examples.

### [Example 1: Isolation of Campanula F3'5'H cDNA]

The two primers CamF1 (5'-GTGAAGCCACCATGTCTATAG-3', SEQ ID NO: 3) and CamR1 (5'-GCATTTGCCTAGACAGTGTAAG-3', SEQ ID NO: 4) were synthesized based on the translated sequence of Campanula *(Campanula medium)* F3'5'H cDNA registered in the DNA database GenBank (Accession No. D14590). The RNA was extracted from commercially available Campanula bud-stage petals using RNeasy Mini Plant Kit (Qiagen), and an RT-PCR kit was used for synthesis of 1st strand DNA. The 1st strand DNA was used as template for PCR using the aforementioned primers.
The obtained DNA fragment was cloned in pCR-TOPO II. Clone #4 (pSPB2561) nucleotide sequence was determined by analysis with a DNA sequencer (SEQ ID NO: 1). While F3'5'H registered as Accession No. D14590 was composed of 523 residues, F3'5'H encoded by clone #4 was composed of 521 residues, and thus exhibited 96% sequence identity with F3'5'H registered as D14590.

### [Reference Example 1: Introduction of pSPB130 into the rose variety "Cool Water" (Constitutive expression of pansy-derived F3'5'H gene and torenia-derived 5AT gene)]

The binary vector pSPB130 described in PTL 3 (a vector constitutively that expresses the pansy F3'5'H gene and torenia anthocyanin 5-acyltransferase gene in plants) was introduced into *Agrobacterium* (Agrobacterium tumefaciens) Agl0. The transformed *Agrobacterium* was introduced into the mauve rose variety "Cool Water" by the method described in PTL 3, to obtain 164 transformants. Anthocyanidin pigment analysis of the petals confirmed delphinidin storage in 51 of the 164 individuals, with a maximum delphinidin content of 76.1% (average: 36.6%).

The analysis values for representative transformants are shown in Table 1 below.

**[Table 1]**

| Plant No. | Delphinidin content (%) | Anthocyanidin (mg/g) | | |
|---|---|---|---|---|
| | | Del | Cya | Pel |
| Cool Water control | 0.0% | 0.0000 | 0.1344 | 0.0005 |
| 1 | 76.1% | 0.0863 | 0.0273 | 0.0000 |
| 2 | 75.7% | 0.1631 | 0.0525 | 0.0000 |
| 3 | 73.9% | 0.0700 | 0.0239 | 0.0008 |
| 4 | 69.8% | 0.0200 | 0.0087 | 0.0000 |
| 5 | 67.1% | 0.1396 | 0.0684 | 0.0000 |
| 6 | 64.4% | 0.0556 | 0.0308 | 0.0000 |
| 7 | 64.4% | 0.0571 | 0.0316 | 0.0000 |
| 8 | 64.0% | 0.1015 | 0.0571 | 0.0000 |
| 9 | 64.0% | 0.1604 | 0.0898 | 0.0004 |
| 10 | 63.3% | 0.0787 | 0.0457 | 0.0000 |

Del: delphinidin, Cya: cyanidin, Pel: pelargonidin Delphinidin content: Proportion of delphinidin in total anthocyanidins.

### [Example 2: Introduction of pSPB2564 into rose variety "Cool Water" (Expression of Campanula-derived F3'5'H gene under the control of E12 35S promoter)]

An expression cassette, comprising the Campanula F3'5'H gene cDNA obtained in Example 1 inserted between the E12 35S promoter described in PTL 3 and the nopaline synthase terminator *(nos* terminator), was inserted into the binary vector pBinPLUS (van Engelen et al. Transgenic Research 4, 288-290, 1995), and the obtained plasmid was designated as pSPB2564. This pSPB2564 was introduced into *Agrobacterium* (Agrobacterium tumefaciens) Agl0. The transformed *Agrobacterium* was used to transform the mauve rose variety "Cool Water" by the method described in PTL 3, to obtain 55 transformants. Anthocyanidin pigment analysis of the petals confirmed delphinidin storage in 48 of the 55 individuals, with a maximum delphinidin content of 97.3% (average: 61.22%).

The analysis values for representative transformants are shown in Table 2 below.

**[Table 2]**

| Plant No. | Delphinidin content (%) | Anthocyanidin (mg/g) | | |
|---|---|---|---|---|
| | | Del | Cya | Pel |
| Cool Water control | 0.0% | 0.0000 | 0.1344 | 0.0005 |
| 1 | 97.3% | 0.1605 | 0.0044 | 0.0000 |
| 2 | 96.1% | 0.2526 | 0.0102 | 0.0000 |
| 3 | 93.0% | 0.5007 | 0.0376 | 0.0000 |
| 4 | 91.7% | 0.0732 | 0.0067 | 0.0000 |
| 5 | 90.3% | 0.1750 | 0.0188 | 0.0000 |
| 6 | 89.0% | 0.1756 | 0.0217 | 0.0000 |
| 7 | 88.9% | 0.1104 | 0.0138 | 0.0000 |
| 8 | 88.7% | 0.1840 | 0.0235 | 0.0000 |
| 9 | 88.5% | 0.0964 | 0.0126 | 0.0000 |
| 10 | 87.0% | 0.1326 | 0.0198 | 0.0000 |

Del: delphinidin, Cya: cyanidin, Pel: pelargonidin Delphinidin content: Proportion of delphinidin in total anthocyanidins.

### [Example 3: Introduction of pSPB2564 into rose variety "Fame" (Expression of Campanula-derived F3'5'H gene under the control of E12 35S promoter)]

The pSPB2564-introduced *Agrobacterium* was used to transform the pink rose variety "Fame", to obtain 114 transformants. Delphinidin storage was confirmed in 46 of the 107 individuals subjected to anthocyanidin pigment analysis of the petals, with a maximum delphinidin content of 98.9% (average: 51.2%).

The analysis values for representative transformants are shown in Table 3 below.

**[Table 3]**

| Plant No. | Delphinidin content (%) | Anthocyanidin (mg/g) | | |
|---|---|---|---|---|
| | | Del | Cya | Pel |
| Fame control | 0.0% | 0.0000 | 0.0704 | 0.0005 |
| 1 | 98.9% | 0.8249 | 0.0092 | 0.0000 |
| 2 | 98.9% | 0.8015 | 0.0091 | 0.0000 |
| 3 | 98.6% | 0.4524 | 0.0063 | 0.0000 |
| 4 | 98.6% | 0.5324 | 0.0076 | 0.0000 |
| 5 | 98.5% | 0.4126 | 0.0063 | 0.0000 |
| 6 | 98.4% | 0.8125 | 0.0133 | 0.0000 |
| 7 | 98.1% | 0.1119 | 0.0022 | 0.0000 |
| 8 | 97.9% | 0.6695 | 0.0142 | 0.0000 |
| 9 | 97.5% | 0.5698 | 0.0148 | 0.0000 |
| 10 | 96.8% | 0.2246 | 0.0073 | 0.0000 |

Del: delphinidin, Cya: cyanidin, Pel: pelargonidin Delphinidin content: Proportion of delphinidin in total anthocyanidins.

### [Example 4: Introduction of pSPB2564 into rose variety "Fame" (Expression of Campanula-derived F3'5'H gene under the control of E12 35S promoter)]

The pSPB2564-introduced *Agrobacterium* was used to transform the pink rose variety "Fame", to obtain 55 transformants. Anthocyanidin pigment analysis of the petals allowed confirmation of delphinidin storage in 28 of the 55 individuals, with a maximum delphinidin content of 98.9% (average: 51.4%).

The analysis values for representative transformants are shown in Table 4 below.

**[Table 4]**

| Plant No. | Delphinidin content (%) | Anthocyanidin (mg/g) | | |
|---|---|---|---|---|
| | | Del | Cya | Pel |
| Fame control | 0.0% | 0.0000 | 0.0704 | 0.0005 |
| 1 | 98.9% | 0.8249 | 0.0092 | 0.0000 |
| 2 | 98.9% | 0.8015 | 0.0091 | 0.0000 |
| 3 | 98.6% | 0.4524 | 0.0063 | 0.0000 |
| 4 | 98.6% | 0.5324 | 0.0076 | 0.0000 |
| 5 | 98.5% | 0.4126 | 0.0063 | 0.0000 |
| 6 | 98.5% | 0.5724 | 0.0090 | 0.0000 |
| 7 | 97.5% | 0.5698 | 0.0148 | 0.0000 |
| 8 | 86.2% | 0.0129 | 0.0021 | 0.0000 |
| 9 | 82.5% | 0.4995 | 0.1061 | 0.0000 |
| 10 | 71.8% | 0.2040 | 0.0797 | 0.0004 |

Del: delphinidin, Cya: cyanidin, Pel: pelargonidin Delphinidin content: Proportion of delphinidin in total anthocyanidins.

### [Example 5: Introduction of pSPB2564 into rose variety "Noblesse" (Expression of Campanula-derived F3'5'H gene under the control of E12 35S promoter)]

The pSPB2564-introduced *Agrobacterium* was used to transform the salmon-pink rose variety "Noblesse", to obtain 3 transformants. Anthocyanidin pigment analysis of the petals allowed confirmation of delphinidin storage in all of the 3 individuals, with a maximum delphinidin content of 98.9% (average: 51.4%).

The analysis values for the transformants are shown in Table 5 below.

**[Table 5]**

| Plant No. | Delphinidin content (%) | Anthocyanidin (mg/g) | | |
|---|---|---|---|---|
| | | Del | Cya | Pel |
| Noblesse control | 0.0% | 0.0000 | 0.0204 | 0.0403 |
| 1 | 94.7% | 0.7076 | 0.0373 | 0.0021 |
| 2 | 92.6% | 0.7966 | 0.0581 | 0.0054 |
| 3 | 90.3% | 0.3333 | 0.0300 | 0.0057 |

Del: delphinidin, Cya: cyanidin, Pel: pelargonidin Delphinidin content: Proportion of delphinidin in total anthocyanidins.

### [Example 6: Introduction of pSPB2564 into rose variety "Ocean Song" (Expression of Campanula-derived F3'5'H gene under the control of E12 35S promoter)]

The pSPB2564-introduced *Agrobacterium* was used to transform the mauve rose variety "Ocean Song", to obtain 40 transformants. Anthocyanidin pigment analysis of the petals allowed confirmation of delphinidin storage in 33 of the 40 individuals, with a maximum delphinidin content of 99.2% (average: 39.7%).

The analysis values for representative transformants are shown in Table 6 below.

**[Table 6]**

| Plant No. | Delphinidin content (%) | Anthocyanidin (mg/g) | | |
|---|---|---|---|---|
| | | Del | Cya | Pel |
| Ocean Song control | 0.0% | 0.0000 | 0.0323 | 0.0000 |
| 1 | 99.2% | 0.1350 | 0.0011 | 0.0000 |
| 2 | 97.3% | 0.0520 | 0.0015 | 0.0000 |
| 3 | 96.9% | 0.0400 | 0.0013 | 0.0000 |
| 4 | 96.6% | 0.1170 | 0.0041 | 0.0000 |
| 5 | 95.3% | 0.0341 | 0.0017 | 0.0000 |
| 6 | 95.0% | 0.0771 | 0.0040 | 0.0000 |
| 7 | 94.7% | 0.0372 | 0.0021 | 0.0000 |
| 8 | 89.8% | 0.0222 | 0.0025 | 0.0000 |
| 9 | 89.6% | 0.0582 | 0.0068 | 0.0000 |
| 10 | 53.5% | 0.0059 | 0.0052 | 0.0000 |

Del: delphinidin, Cya: cyanidin, Pel: pelargonidin Delphinidin content: Proportion of delphinidin in total anthocyanidins.

### [Example 7: Introduction of pSPB2564 into rose variety "Peach Avalanche" (Expression of Campanula-derived F3'5'H gene under the control of E12 35S promoter)]

The pSPB2564-introduced *Agrobacterium* was used to transform the light salmon-pink rose variety "Peach Avalanche", to obtain 11 transformants. Anthocyanidin pigment analysis of the petals allowed confirmation of delphinidin storage in 1 of the 11 individuals, with a maximum delphinidin content of 99.3%.

The analysis values for the transformants in which delphinidin production was confirmed are shown in Table 7 below.

**[Table 7]**

| Plant No. | Delphinidin content (%) | Anthocyanidin (mg/g) | | |
|---|---|---|---|---|
| | | Del | Cya | Pel |
| Peach Avalanche control | 0.0% | 0.0000 | 0.0009 | 0.0009 |
| 1 | 99.3% | 0.3156 | 0.0021 | 0.0000 |

Del: delphinidin, Cya: cyanidin, Pel: pelargonidin Delphinidin content: Proportion of delphinidin in total anthocyanidins.

### [Example 8: Introduction of pSPB2564 into rose variety "Topless" (Expression of Campanula-derived F3'5'H gene under the control of E12 35S promoter)]

The pSPB2564-introduced *Agrobacterium* was used to transform the salmon-pink rose variety "Topless", to obtain 11 transformants. Anthocyanidin pigment analysis of the petals allowed confirmation of delphinidin storage in 10 of the 11 individuals, with a maximum delphinidin content of 99.4% (average: 64.4%).

The analysis values for the transformants in which delphinidin production was confirmed are shown in Table 8 below.

**[Table 8]**

| Plant No. | Delphinidin content (%) | Anthocyanidin (mg/g) | | |
|---|---|---|---|---|
| | | Del | Cya | Pel |
| Topless control | 0.0% | 0.0000 | 0.0341 | 0.2721 |
| 1 | 99.4% | 0.9456 | 0.0057 | 0.0000 |
| 2 | 99.4% | 1.4935 | 0.0097 | 0.0000 |
| 3 | 99.2% | 2.2857 | 0.0181 | 0.0000 |
| 4 | 99.1% | 1.6183 | 0.0152 | 0.0000 |
| 5 | 89.3% | 1.0868 | 0.1007 | 0.0297 |
| 6 | 42.2% | 0.1676 | 0.0724 | 0.1576 |
| 7 | 42.2% | 0.1486 | 0.0839 | 0.1197 |
| 8 | 36.3% | 0.2109 | 0.1996 | 0.1712 |
| 9 | 36.0% | 0.1341 | 0.1184 | 0.1202 |
| 10 | 0.6% | 0.0016 | 0.1182 | 0.1665 |

Del: delphinidin, Cya: cyanidin, Pel: pelargonidin Delphinidin content: Proportion of delphinidin in total anthocyanidins.

### [Reference Example 2: Introduction of pSPB5202 into rose variety "Fame" (Expression of Campanula-derived F3'5'H gene under the control of pansy-derived F3'5'H promoter)]

The pansy-derived F3'5'H promoter sequence obtained by digesting plasmid pSFL620 described in PTL 9 with HindIII and PstI and the Campanula cDNA sequence obtained in Example 1 were linked with heat shock protein terminator from plasmid pRI201-AN (Takara Bio, Inc.) and inserted into binary vector pBinPlus. The obtained binary vector was designated as pSPB5202. This pSPB5202 was introduced into *Agrobacterium* (Agrobacterium tumefaciens) Agl0. The transformed *Agrobacterium* was used to transform the pink rose variety "Fame" by the method described in PTL 3, to obtain 56 transformants. Delphinidin storage could be confirmed in only 4 of the 52 individuals subjected to anthocyanidin pigment analysis of the petals, with a maximum delphinidin content of 10.8% (average: 3.9%).

The analysis values for representative transformants are shown in Table 9 below.

**[Table 9]**

| Plant No. | Delphinidin content (%) | Anthocyanidin (mg/g) | | |
|---|---|---|---|---|
| | | Del | Cya | Pel |
| Fame control | 0.0% | 0.0000 | 0.0704 | 0.0005 |
| 1 | 10.8% | 0.0126 | 0.1049 | 0.0000 |
| 2 | 2.9% | 0.0028 | 0.0920 | 0.0010 |
| 3 | 1.4% | 0.0011 | 0.0756 | 0.0005 |
| 4 | 0.5% | 0.0000 | 0.1220 | 0.0006 |
| 5 | 0.0% | 0.0000 | 0.0110 | 0.0005 |
| 6 | 0.0% | 0.0000 | 0.0163 | 0.0006 |
| 7 | 0.0% | 0.0000 | 0.0625 | 0.0000 |
| 8 | 0.0% | 0.0000 | 0.0173 | 0.0008 |
| 9 | 0.0% | 0.0000 | 0.0068 | 0.0003 |
| 10 | 0.0% | 0.0000 | 0.0466 | 0.0013 |

Del: delphinidin, Cya: cyanidin, Pel: pelargonidin Delphinidin content: Proportion of delphinidin in total anthocyanidins.

Although the pansy-derived F3'5'H promoter satisfactorily expresses the pansy-derived F3'5'H gene in rose (see PTL 9) and heat shock protein terminator is very effective for expressing foreign genes in plants (Plant Cell Physiol (2010) 51, 328-332), only very low levels of delphinidin production was found in the transformed rose petals, suggesting that the Campanula F3'5'H gene did not function sufficiently. This indicates that in order to efficiently produce delphinidin in specific rose varieties, it is necessary to select a combination of a specific promoter and a specific F3'5'H gene, or a combination of a specific promoter, a specific F3'5'H gene and a specific terminator gene.

### [Reference Example 3: Introduction of pSPB5204 into rose variety "Fame" (Expression of Campanula-derived F3'5'H gene under the control of perilla-derived 3AT promoter)]

Upon digesting plasmid pSFL205 described in PTL 10 with HindIII and BamHI, an approximately 1.1 kb DNA fragment comprising the perilla-derived anthocyanin 3-acyltransferase promoter portion was recovered, and this was linked with pBinPlus digested with HindIII and BamHI to obtain pSPB3756. Also, in PCR using plasmid pSPB3311 described in PTL 10 as template with the three different synthetic nucleotide primers: Pf3AT-FW-Sal (5'-ATGTCGACTAAATGTATGTAATTAAC-3', SEQ ID NO: 5), Pf3ATt-R1 (5'-ACTCAACACTTTATTAATTG-3', SEQ ID NO: 6) and Pf3ATt-F1 (5'-ATGTAACAATTAATTAAGTG-3', SEQ ID NO: 7), the restriction enzyme SalI recognition sequence was added at the 5'-end of the perilla-derived anthocyanin acyltransferase terminator portion, and the PacI recognition site at the 5'-end in the PacI recognition site in the same terminator portion was disrupted. The DNA fragment obtained in PCR was cloned in pTOPO. A DNA fragment obtained by digesting the obtained plasmid with KpnI and PacI was linked with a DNA fragment obtained by digesting pSPB3756 with KpnI and PacI, to obtain pSPB5201. Plasmid pSPB5201 digested with SmaI and SpeI was linked with the Campanula cDNA obtained in Example 1, and the obtained binary vector was designated as pSPB5204. This pSPB5204 was introduced into *Agrobacterium* (Agrobacterium tumefaciens) Agl0. The transformed *Agrobacterium* was used to transform the pink rose variety "Fame" by the method described in PTL 3, to obtain 63 transformants. Delphinidin storage could be confirmed in only 8 of the 61 individuals subjected to anthocyanidin pigment analysis of the petals, with a maximum delphinidin content of 2.8% (average: 1.3%).

The analysis values for representative transformants are shown in Table 10 below.

**[Table 10]**

| Plant No. | Delphinidin content (%) | Anthocyanidin (mg/g) | | |
|---|---|---|---|---|
| | | Del | Cya | Pel |
| Fame control | 0.0% | 0.0000 | 0.0704 | 0.0005 |
| 1 | 2.8% | 0.0016 | 0.0542 | 0.0004 |
| 2 | 2.0% | 0.0022 | 0.1056 | 0.0007 |
| 3 | 1.2% | 0.0000 | 0.0582 | 0.0005 |
| 4 | 1.2% | 0.0011 | 0.0935 | 0.0006 |
| 5 | 1.1% | 0.0004 | 0.0349 | 0.0004 |
| 6 | 0.9% | 0.0011 | 0.1231 | 0.0006 |
| 7 | 0.7% | 0.0008 | 0.1037 | 0.0007 |
| 8 | 0.2% | 0.0002 | 0.0745 | 0.0004 |
| 9 | 0.0% | 0.0000 | 0.0521 | 0.0012 |
| 10 | 0.0% | 0.0000 | 0.0417 | 0.0000 |

Del: delphinidin, Cya: cyanidin, Pel: pelargonidin Delphinidin content: Proportion of delphinidin in total anthocyanidins.

Although the perilla-derived anthocyanin 3-acyltransferase promoter satisfactorily expressed the perilla-derived anthocyanin 3-acyltransferase gene in rose, petunia and chrysanthemum (see PTL 9), only very low levels of delphinidin were produced in the rose petals of these transformants, suggesting that the Campanula F3'5'H gene did not sufficiently function.
This indicates that in order to efficiently produce delphinidin in rose, it is necessary to select a combination of a specific promoter and a specific F3'5'H gene, or a combination of a specific promoter, a specific F3'5'H gene and a specific terminator gene.

### Industrial Applicability

By transformation using a plasmid containing the Campanula flavonoid 3',5'-hydroxylase gene of the invention under the control of the cauliflower mosaic virus 35S promoter by the *Agrobacterium* method, it is possible to notably increase the delphinidin content in the petals of specific varieties of rose plants. Thus, the invention can be suitably used for production of rose plants with altered flower color.

## Claims

1. A polynucleotide selected from the group consisting of the following (a) to (d):
(a) a polynucleotide comprising the nucleotide sequence listed as SEQ ID NO: 1;
(b) a polynucleotide having at least 97% sequence identity with a polynucleotide comprising the nucleotide sequence listed as SEQ ID NO: 1 or its complementary nucleotide sequence, and encoding a protein having flavonoid 3',5'-hydroxylase activity,
(c) a polynucleotide encoding a protein comprising the amino acid sequence listed as SEQ ID NO: 2; and
(d) a polynucleotide encoding a protein having an amino acid sequence with at least 97% sequence identity with the amino acid sequence listed as SEQ ID NO: 2, and having flavonoid 3',5'-hydroxylase activity.

2. A plasmid in which the polynucleotide according to claim 1 is linked in a functional manner under the control of the cauliflower mosaic virus 35S promoter.

3. A plasmid according to claim 2, wherein the *nos* terminator is further linked in a functional manner.

4. A method for producing a transformed rose plant or its progeny having a delphinidin content of 80% or greater in the petals, by transformation of a garden rose variety *(Rosa hybrida*) by the *Agrobacterium* method using a plasmid according to claim 2 or 3.

5. The method according to claim 4, wherein the delphinidin content in the petals is 85% or greater.

6. The method according to claim 5, wherein the delphinidin content in the petals is 90% or greater.

7. The method according to claim 6, wherein the delphinidin content in the petals is 95% or greater.

8. A transformed rose plant or its progeny, produced by the method according to any one of claims 4 to 7.


### [Sequence Listing]

SEQUENCE LISTING

<110>  Suntory Holdings Limited

<120>  Novel Campanula F3'5'H gene and use thereof

<130>  SP2012-0132(AB587)

<150>  JP2012-092716
<151>  2012-04-16

<160>  7

<170>  PatentIn version 3.5

<210>  1
<211>  1585
<212>  DNA
<213>  Campanula medium

<220>
<221>  misc_feature
<223>  F3'5'H Clone 4

<220>
<221>  CDS
<222>  (12)..(1574)

<400>  1
gtgaagccac c atg tct ata gac ata acc att ctc tta tgt gaa ctt gtt      50
             Met Ser Ile Asp Ile Thr Ile Leu Leu Cys Glu Leu Val
             1               5                   10

gct gca att tca ctc tac tta tta acc tac tat ttc att tgt ttc ctc      98
Ala Ala Ile Ser Leu Tyr Leu Leu Thr Tyr Tyr Phe Ile Cys Phe Leu
    15                  20                  25

ttc aaa ccc tct cat cat cac cac cac ctc cct ccc ggc cca acc gga      146
Phe Lys Pro Ser His His His His His Leu Pro Pro Gly Pro Thr Gly
30                  35                  40                  45

tgg ccg atc att gga tcc ctt cct ctc tta ggc act atg cca cat gtt      194
Trp Pro Ile Ile Gly Ser Leu Pro Leu Leu Gly Thr Met Pro His Val
                50                  55                  60

tcc tta gcc gac atg gcc gta aaa tac ggg cct ata atg tac cta aaa      242
Ser Leu Ala Asp Met Ala Val Lys Tyr Gly Pro Ile Met Tyr Leu Lys
            65                  70                  75

ctt ggt tca aag ggc acc gtc gtg gcc tca aat cca aaa gcc gcc cga      290
Leu Gly Ser Lys Gly Thr Val Val Ala Ser Asn Pro Lys Ala Ala Arg
        80                  85                  90

gca ttc ttg aaa tcc cat gat gcc aat ttt tct aac cgt ccg att gat      338
Ala Phe Leu Lys Ser His Asp Ala Asn Phe Ser Asn Arg Pro Ile Asp
    95                  100                 105

ggg ggg ccc acc tac ctc gcg tat aat gca caa gac atg gtt ttt gca      386
Gly Gly Pro Thr Tyr Leu Ala Tyr Asn Ala Gln Asp Met Val Phe Ala
110                 115                 120                 125

gaa tat ggc cca aaa tgg aag ctt ttg cga aag cta tgt agc ttg cac      434

Glu Tyr Gly Pro Lys Trp Lys Leu Leu Arg Lys Leu Cys Ser Leu His
                130                 135                 140

atg tta ggc ccg aag gca ctc gag gat tgg gct cat gtc aga gtt tca      482
Met Leu Gly Pro Lys Ala Leu Glu Asp Trp Ala His Val Arg Val Ser
            145                 150                 155

gag gtc ggt cat atg ctc aaa gaa atg tac gag caa tcg agt aag tcc      530
Glu Val Gly His Met Leu Lys Glu Met Tyr Glu Gln Ser Ser Lys Ser
        160                 165                 170

gtg cca gtg gtg gtg cca gag atg tta act tat gcc atg gct aat atg      578
Val Pro Val Val Val Pro Glu Met Leu Thr Tyr Ala Met Ala Asn Met
    175                 180                 185

att gga cga atc ata ctc agt cga cgc cct ttt gtt atc acg agc aaa      626
Ile Gly Arg Ile Ile Leu Ser Arg Arg Pro Phe Val Ile Thr Ser Lys
190                 195                 200                 205

tta gac tcg tct gct tct gct gct tct gtt agt gaa ttc caa tat atg      674
Leu Asp Ser Ser Ala Ser Ala Ala Ser Val Ser Glu Phe Gln Tyr Met
                210                 215                 220

gtt atg gag ctc atg agg atg gca ggg ttg ttc aat att ggt gat ttc      722
Val Met Glu Leu Met Arg Met Ala Gly Leu Phe Asn Ile Gly Asp Phe
            225                 230                 235

ata cca tat att gcg tgg atg gat ttg caa ggc att caa cgc gat atg      770
Ile Pro Tyr Ile Ala Trp Met Asp Leu Gln Gly Ile Gln Arg Asp Met
        240                 245                 250

aag gtt ata cag caa aag ttt gat gtc ttg ttg aac aaa atg atc aag      818
Lys Val Ile Gln Gln Lys Phe Asp Val Leu Leu Asn Lys Met Ile Lys
    255                 260                 265

gaa cat aca gaa tcc gct cat gat cgt aaa gat aat cct gat ttt ctt      866
Glu His Thr Glu Ser Ala His Asp Arg Lys Asp Asn Pro Asp Phe Leu
270                 275                 280                 285

gat att ctt atg gcg gct acc caa gaa aac acg gag gga att cag ctt      914
Asp Ile Leu Met Ala Ala Thr Gln Glu Asn Thr Glu Gly Ile Gln Leu
                290                 295                 300

aat ctc gta aat gtt aag gcg ctt ctt ttg gat tta ttc acg gcg ggc      962
Asn Leu Val Asn Val Lys Ala Leu Leu Leu Asp Leu Phe Thr Ala Gly
            305                 310                 315

acg gat aca tcg tcg agt gtg atc gaa tgg gca cta gcc gaa atg ttg     1010
Thr Asp Thr Ser Ser Ser Val Ile Glu Trp Ala Leu Ala Glu Met Leu
        320                 325                 330

aac aat cga cag atc cta aac cgg gcc cac gaa gaa atg gac caa gtc     1058
Asn Asn Arg Gln Ile Leu Asn Arg Ala His Glu Glu Met Asp Gln Val
    335                 340                 345

att ggc aga aac aga aga cta gaa caa tct gac ata cca aac ttg cca     1106
Ile Gly Arg Asn Arg Arg Leu Glu Gln Ser Asp Ile Pro Asn Leu Pro
350                 355                 360                 365

tat ttc caa gcc ata tgc aaa gaa aca ttc cga aaa cac cct tcc acg     1154
Tyr Phe Gln Ala Ile Cys Lys Glu Thr Phe Arg Lys His Pro Ser Thr
                370                 375                 380

ccc tta aac ctc cca aga atc tca aca gaa gaa tgt gaa gtc gaa gga     1202
Pro Leu Asn Leu Pro Arg Ile Ser Thr Glu Glu Cys Glu Val Glu Gly
            385                 390                 395

ttt cgc ata ccc aaa aac act aga cta ata gtg aac ata tgg gca ata     1250
Phe Arg Ile Pro Lys Asn Thr Arg Leu Ile Val Asn Ile Trp Ala Ile
        400                 405                 410

ggg aga gac cct aaa gtg tgg gaa aat cca ttg gat ttt acc ccg gaa     1298
Gly Arg Asp Pro Lys Val Trp Glu Asn Pro Leu Asp Phe Thr Pro Glu
    415                 420                 425

cga ttc ttg agt gaa aaa cac gcg aaa att gat ccg cga ggt aat cat     1346
Arg Phe Leu Ser Glu Lys His Ala Lys Ile Asp Pro Arg Gly Asn His
430                 435                 440                 445

ttt gag tta atc cca ttt ggg gcg gga cgg agg ata tgt gca ggg gct     1394
Phe Glu Leu Ile Pro Phe Gly Ala Gly Arg Arg Ile Cys Ala Gly Ala
                450                 455                 460

aga atg gga gcg gcc tcg gtc gag tac att tta ggt aca ttg gtg cac     1442
Arg Met Gly Ala Ala Ser Val Glu Tyr Ile Leu Gly Thr Leu Val His
            465                 470                 475

tca ttt gat tgg aaa ttg cct gat gga gtt gtg gaa gtt aat atg gaa     1490
Ser Phe Asp Trp Lys Leu Pro Asp Gly Val Val Glu Val Asn Met Glu
        480                 485                 490

gag agc ttt ggg ata gca ttg cag aaa aag atg cct ctt tct gct att     1538
Glu Ser Phe Gly Ile Ala Leu Gln Lys Lys Met Pro Leu Ser Ala Ile
    495                 500                 505

gtt act cca aga ttg cct cca agt gct tac act gtc taggcaaatg c        1585
Val Thr Pro Arg Leu Pro Pro Ser Ala Tyr Thr Val
510                 515                 520


<210>  2
<211>  521
<212>  PRT
<213>  Campanula medium

<400>  2

Met Ser Ile Asp Ile Thr Ile Leu Leu Cys Glu Leu Val Ala Ala Ile
1               5                   10                  15


Ser Leu Tyr Leu Leu Thr Tyr Tyr Phe Ile Cys Phe Leu Phe Lys Pro
            20                  25                  30


Ser His His His His His Leu Pro Pro Gly Pro Thr Gly Trp Pro Ile
        35                  40                  45


Ile Gly Ser Leu Pro Leu Leu Gly Thr Met Pro His Val Ser Leu Ala
    50                  55                  60


Asp Met Ala Val Lys Tyr Gly Pro Ile Met Tyr Leu Lys Leu Gly Ser
65                  70                  75                  80

Lys Gly Thr Val Val Ala Ser Asn Pro Lys Ala Ala Arg Ala Phe Leu
                85                  90                  95

Lys Ser His Asp Ala Asn Phe Ser Asn Arg Pro Ile Asp Gly Gly Pro
            100                 105                 110

Thr Tyr Leu Ala Tyr Asn Ala Gln Asp Met Val Phe Ala Glu Tyr Gly
        115                 120                 125

Pro Lys Trp Lys Leu Leu Arg Lys Leu Cys Ser Leu His Met Leu Gly
    130                 135                 140

Pro Lys Ala Leu Glu Asp Trp Ala His Val Arg Val Ser Glu Val Gly
145                 150                 155                 160

His Met Leu Lys Glu Met Tyr Glu Gln Ser Ser Lys Ser Val Pro Val
                165                 170                 175

Val Val Pro Glu Met Leu Thr Tyr Ala Met Ala Asn Met Ile Gly Arg
            180                 185                 190

Ile Ile Leu Ser Arg Arg Pro Phe Val Ile Thr Ser Lys Leu Asp Ser
        195                 200                 205

Ser Ala Ser Ala Ala Ser Val Ser Glu Phe Gln Tyr Met Val Met Glu
    210                 215                 220

Leu Met Arg Met Ala Gly Leu Phe Asn Ile Gly Asp Phe Ile Pro Tyr
225                 230                 235                 240

Ile Ala Trp Met Asp Leu Gln Gly Ile Gln Arg Asp Met Lys Val Ile
                245                 250                 255

Gln Gln Lys Phe Asp Val Leu Leu Asn Lys Met Ile Lys Glu His Thr
            260                 265                 270

Glu Ser Ala His Asp Arg Lys Asp Asn Pro Asp Phe Leu Asp Ile Leu
        275                 280                 285

Met Ala Ala Thr Gln Glu Asn Thr Glu Gly Ile Gln Leu Asn Leu Val
    290                 295                 300

Asn Val Lys Ala Leu Leu Leu Asp Leu Phe Thr Ala Gly Thr Asp Thr
305                 310                 315                 320

Ser Ser Ser Val Ile Glu Trp Ala Leu Ala Glu Met Leu Asn Asn Arg
                325                 330                 335

Gln Ile Leu Asn Arg Ala His Glu Glu Met Asp Gln Val Ile Gly Arg
            340                 345                 350

Asn Arg Arg Leu Glu Gln Ser Asp Ile Pro Asn Leu Pro Tyr Phe Gln
        355                 360                 365

Ala Ile Cys Lys Glu Thr Phe Arg Lys His Pro Ser Thr Pro Leu Asn
    370                 375                 380

Leu Pro Arg Ile Ser Thr Glu Glu Cys Glu Val Glu Gly Phe Arg Ile
385                 390                 395                 400

Pro Lys Asn Thr Arg Leu Ile Val Asn Ile Trp Ala Ile Gly Arg Asp
                405                 410                 415

Pro Lys Val Trp Glu Asn Pro Leu Asp Phe Thr Pro Glu Arg Phe Leu
            420                 425                 430

Ser Glu Lys His Ala Lys Ile Asp Pro Arg Gly Asn His Phe Glu Leu
        435                 440                 445

Ile Pro Phe Gly Ala Gly Arg Arg Ile Cys Ala Gly Ala Arg Met Gly
    450                 455                 460

Ala Ala Ser Val Glu Tyr Ile Leu Gly Thr Leu Val His Ser Phe Asp
465                 470                 475                 480

Trp Lys Leu Pro Asp Gly Val Val Glu Val Asn Met Glu Glu Ser Phe
                485                 490                 495

Gly Ile Ala Leu Gln Lys Lys Met Pro Leu Ser Ala Ile Val Thr Pro
            500                 505                 510

Arg Leu Pro Pro Ser Ala Tyr Thr Val
        515                 520

<210>  3
<211>  21
<212>  DNA
<213>  Artificial

<220>
<223>  Primer CamF1

<400>  3
gtgaagccac catgtctata g                                                21

<210>  4

<211>  22
<212>  DNA
<213>  Artificial

<220>
<223>  Primer CamR1

<400>  4
gcatttgcct agacagtgta ag                                              22


<210>  5
<211>  26
<212>  DNA
<213>  Artificial Sequence

<220>
<223>  primer Pf3AT FW Sal

<400>  5
atgtcgacta aatgtatgta attaac                                          26


<210>  6
<211>  20
<212>  DNA
<213>  Artificial Sequence

<220>
<223>  primer Pf3ATt R1

<400>  6
actcaacact ttattaattg                                                 20


<210>  7
<211>  20
<212>  DNA
<213>  Artificial Sequence

<220>
<223>  primer Pf3ATt F1

<400>  7
atgtaacaat taattaagtg                                                 20